# EUROPEAN PATENT APPLICATION

(11) **EP 0 753 304 A1**
(43) Date of publication of application: **15.01.1997**
(21) Application number: 95920321.7
(22) Date of filing: 25.05.1995
(51) Int. Cl.: A61K 35/62, A61K 37/02

(54) **THROMBOLYTIC ENZYME AND METHOD OF OBTAINING SAME**

(30) Priority: 23.12.1994 RU 94043698
(71) Applicant: INSTITUT BIOORGANICHESKOI KHIMII IM. M.M. SHEMYAKINA I JU. A.OVCHINNIKOVA RAN, Moscow, 117871 (SU)
(72) Inventor: SVERDLOV, Evgeny Davidovich, Moscow, 119517 (RU); BASKOVA, Izolda Porfirievna, Moscow, 123100 (RU); ZAVALOVA, Lyudmila Lvovna, Moscow, 117296 (RU); LUKYANOV, Sergei Anatolievich, Moscow, 117574 (RU); BARSOVA, Ekaterina Vladimirovna, Moscow, 113570 (RU); BOGDANOVA, Ekaterina Andreevna, Moscow, 117465 (RU)
(74) Representative: Zellentin, Rüdiger
(86) International application number: RU9500102
(87) International publication number: WO9619999

(57) **Abstract**

A thrombolytic enzyme capable of hydrolyzing exo-and endo - ε - (γ - Glu) - Lys - isopeptide bonds in synthetic substrates, in stabilized fibrin and in its D-D-fragment with a partial amino acid sequence with a structure of a corresponding thereto gene and two additional genes of that same family, with residues of glycine in positions 15, 21, 24, 28, 33, 46, 49, 50 in the primary structure of the protein.

A method of preparing a thrombolytic enzyme from medicinal leech salivary gland secretion, or preparations comprising that secretion, including affinity chromatography on antibodies to native enzyme which are immobilized on a solid carrier, subsequent purification of the enzyme by ion-exchange chromatography and gel filtration.

## Description

### Field of the Invention

The invention in the field of medicine relates to hemostasiology, pharmacology and is designed for use in clinical and experimental medicine as a thrombolytic biologically active preparation.

### Prior Art

At present the use of medicinal leech salivary gland secretion remains in the row of various sources used for the preparation of biologically active substances possessing thrombolytic, fibrinolytic activity, which is being filled in. A practical interest in the native secretion of the medicinal leech, which is a source of biologically active substances, is due to the determination of the dependence of the efficiency of treatment of thrombophlebitis of different etiology when thrombolytic effect of medicinal leech is used during the leeching in hirudotherapy (Zaitsev G.B. "Thrombophlebitis," 1947, Medgiz, Moscow, p. 78).

Use of medicinal leech salivary gland secretion is due to the discovery of a new mechanism for conversion into a new quality.

It was shown during an analysis of the properties of medicinal leech salivary gland secretion that it is almost completely free of proteolytic activity when used as substrates casein and non-stabilized fibrin. However, during the incubation with insoluble stabilized fibrin, cross-linked with factor XIIIa, it is capable of converting it into a soluble state, and to a greater degree the higher the degree of stabilization of fibrin, i.e. the more ε - (γ - Glu) - Lys - isopeptide bonds there are in the stabilized fibrin preparation. This, at first sight paradoxical situation, was explained by us to be due to the presence on an enzyme in the composition of a leech secretion, specifically hydrolyzing isopeptide bonds in stabilized fibrin and not acting on the non-stabilized fibrin in which these isopeptide bonds are absent. This enzyme was called destabilase (Biokhimiya, 50, 1985, Nauka, Moscow, pp. 424-431).

Known thrombolytic and fibrinolytic enzymes catalyze the hydrolysis of peptide bonds in fibrin independent of the degree of "cross-linking" fibrin with ε - (γ - Glu) - Lys - isopeptide bonds which are formed as a result of the action of the enzyme of transpeptidase, factor XIIIa. Usually fibrin "linked" with isopeptide bonds or stabilized is more tolerant to the effect of proteolytic enzymes than non-stabilized fibrin. At the same time non-stabilized fibrin is capable of passing into a soluble state not only as a result of proteolysis stimulated by fibrinolytic enzymes, but also under the effect of nonspecific agents, weakening ionic, hydrophobic and H - O - interaction between fibrin monomers.

### Disclosure of the Invention

The invention is directed to the disclosure of a substance, its properties and a method of preparing both a thrombolytic enzyme having amidolytic exo-isopeptidase and endo-isopeptidase activity, with the capability of dissolving thrombi, and distinguished over known preparations obtained from the medicinal leech by molecular mass and amino acid sequence.

The stated method of preparing an enzyme of destabilase is carried out in the following manner.

An extract from the medicinal leeches is subjected to affinity chromatography which is carried out on a column with immobilized on sepharose 4B antibodies to destabilase. The column is equilibrated by a 0.02 M tris-HCl buffer solution, pH = 7.4. The unlinked protein is washed with a buffer solution and the destabilase is eluted with the same buffer solution comprising 0.3 M NaCl. The obtained preparation is again chromatographed on a column with a CM-trisacrylic sorbent, equilibrated with a 0.02 M tris-HCl buffer solution, pH = 7.4. The desired product is eluted with 0.05 M - 0.20 M NaCl dissolved in the same 0.02 M tris-HCl buffer solution. Fractions comprising the final product are pooled, desalted by gel filtration on Superose-12 and freeze dried. As a result the obtained preparation comprises 100% active substance. The degree of purity is confirmed electrophoretically.

Primary structure. In order to identify the primary structure, the enzyme is chromatographed on a reverse-phase column with Aquapore RP-300 (4.6 x 100) in a gradient of acetonitrile from 0 to 60% for 60 min. The N-terminal sequence of destabilase is determined. Threonine is determined as the N-terminal amino acid. Then CNBr hydrolysis of the destabilase is carried out and the obtained mixture is again chromatographed on the same column under the same conditions. As a result four peptide fragments are obtained. The amino acid sequence of the first fragment is determined, it consists of 25 amino acid residues and has a residue of threonine at the NH₂-terminal: Thr Val Pro Ser Asp Cys Leu Arg Cye Ile Cys Gln Val Glu Gly Cys Asn Asn Glu Ile Gly Arg Cys Gly Met.

Amino acid sequence of 28 amino acids from the N-terminal, with aspartic acid at the NH₂-terminal, has been determined for the second fragment: Asp Ala Gly Ser Leu Ser Cys Gly Pro Tyr Gln Ile Lys Glu Pro Tyr X Ile Asp X Gly Arg Pro Gly Gly X Tyr Gln.

Amino acid sequence of 24 amino acid residues from the N-terminal, with aspartic acid at the NH₂-terminal, has been determined for the third fragment: Asp Arg Tyr Ala Pro Pro Cys Thr Gly Gly Arg Gln Pro Thr Cys Gln Asp Tyr Ala Lys Ile His Asn Met.

A partial sequence of 10 amino acid residues from the N-terminal has been determined for the fourth fragment: Gly Pro Asn Gly Cys Gln Ser Leu Asn Asn.

Glycine is the N-terminal amino acid.

Using standard processes, m-RNA was isolated from the medicinal leech. The first c-DNA chain was synthesized on its base using reverse transcription. Amplification of the DNA fragment encoding destabilase was brought into a polymeric chain reaction using the first c-DNA chain obtained at the preceding stage and primers selected on the base of the amino acid sequence of destabilase. As a result a DNA fragment of predicted length--102 nucleotide pairs--is obtained. Cloning and sequencing of the obtained DNA fragment were carried out in accordance with standard methods (Maniatia et al., 1982; Tabor, Richardson, 1987). As a result of sequencing 10 independent clones, two different DNA sequences--DS1 and DS2--have been detected. The differences in the nucleotide sequence of DS1 and DS2 have made it possible to come to the conclusion that there is a family of genes in the leech genom which encode the affined proteins.

The partial sequence DS1 and the full nucleotide sequence DS2 are presented below.

Nucleotide sequence DS1:
leader:
ATG AAT TAC GTT ATC TTT GTG GTC TTA GTG GCA CTT TAC GTC 14
Met Asn Tyr Val Ile Phe Val Val Leu Val Ala Leu Tyr Val

ATC GAC GTA GCG AAG TGC 20
Ile Asp Val Ala Lys Cys

structural part:

ACC GTT CCA TCC GAC TGC TTG AGT TGC ATT TGC GAG GTA GAG 34
Thr Val Pro Ser Asp Cys Leu Ser Cys Ile Cys Glu Val Glu

GGA TGT GAC AAA GAG ATT GGA AGG TGC GGC GAT GAC GCA GGA 48
Gly Cys Asp Lys Glu Ile Gly Arg Cys Gly Asp Asp Ala Gly

AGT CTG AGC TGT GGT CCT ...
Ser Leu Ser Cys Gly Pro ...

Nucleotide sequence DS2:
leader:
ATG ATC ATT GCA ATT TAT GTT TCC CTA GCT CTT CTA ATC GCC 14
Met Ile Ile Ala Ile Tyr Val Ser Leu Ala Leu Leu Ile Ala

TCT GTC GAG GTG AAT AGC 20
Ser Val Glu Val Asn Ser

structural part:
CAA TTC ACT GAT TCT TGC CTT CGG TGT ATT TGC AAG GTG GAA 34
Gln Phe Thr Asp Ser Cys Leu Arg Cys Ile Cys Lys Val Glu

GGA TGT GAC AGT CAA ATT GGA AAA TGT GGA ATG GAT GTT GGA 48
Gly Cys Asp Ser Gln Ile Gly Lys Cys Gly Met Asp Val Gly

AGC TTG AGT TGC GGA CCA TAC CAG ATT AAG AAA CCG TAG TGG 62
Ser Leu Ser Cys Gly Pro Tyr Gln Ile Lys Lys Pro Tyr Trp

ATT GAT TGT GGA AAA CCA GGG GGA GGT TAC GAA TCA TGC ACA 76
Ile Asp Cys Gly Lys Pro Gly Gly Gly Tyr Glu Ser Cys Thr

AAA AAT AAA GCC TGT TGA GAG ACT TGT GTG AGA GCT TAC ATG 90
Lys Asn Lys Ala Cys Ser Glu Thr Cys Val Arg Ala Tyr Met

AAG AGG TAT GGA ACC TTC TGC ACA GGT GGA CGA ACC CCA ACC 104
Lys Arg Tyr Gly Thr Phe Cys Thr Gly Gly Arg Thr Pro Thr

TGC CAG GAT TAT GCT AGG ATT CAT AAC GGT GGA CCA CGC GGT 118
Cys Gln Asp Tyr Ala Arg Ile His Asn Gly Gly Pro Arg Gly

TGC AAG AGT TCT GCT ACT GTT GGT TAC TGG AAC AAG GTA CAG 132
Cys Lys Ser Ser Ala Thr Val Gly Tyr Trp Asn Lys Val Gln

AAA TGT TTG AGA 136
Lys Cys Leu Arg

### Best Method of Carrying Out the Invention

The method of preparing a enzyme destabilase is illustrated by the following concrete examples of its implementation.

### Example 1.

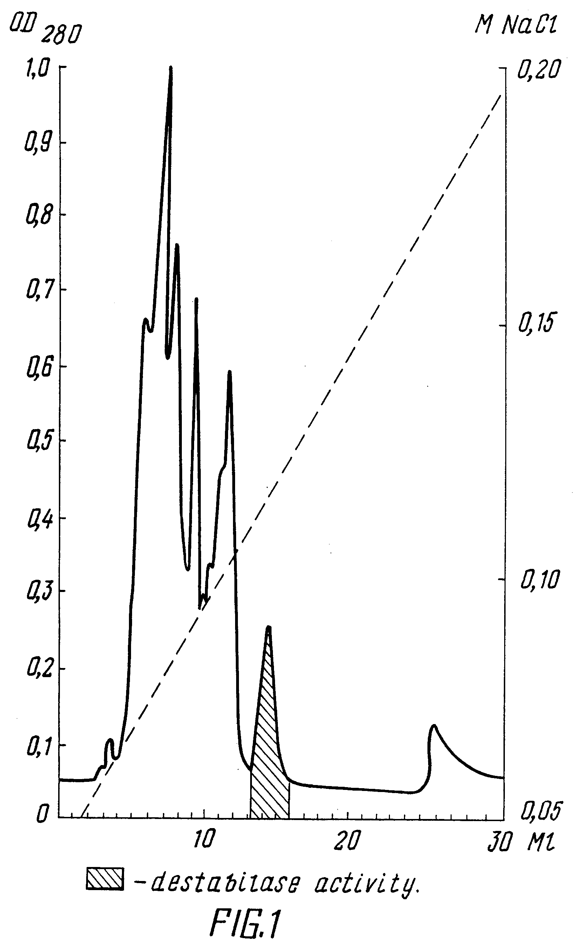

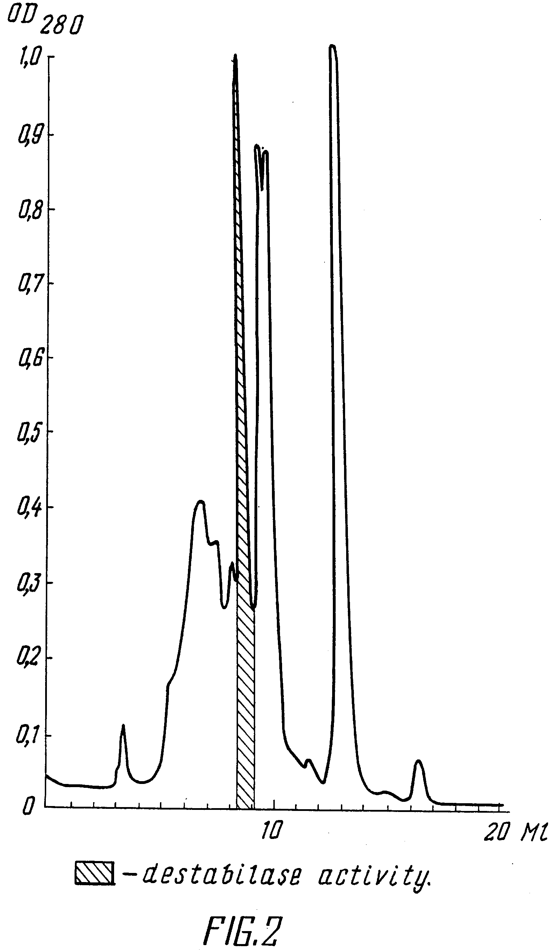

Medicinal leeches, starved for at least three months, are passed through a meat-grinder and extracted with physiological solution. The extract is applied to a column with sepharose 4B with immobilized thereon antibodies to destabilase, equilibrated with a 0.02 M tris-HCl buffer solution, pH = 7.4. The enzyme is eluted with 0.3 M NaCl dissolved in a 0.02 M tris-HCl buffer solution, pH = 7.4. The fractions are pooled. The obtained solution (8 ml, protein concentration 0.5 mg/ml) is applied to a column with a CM-trisacrylic sorbent, equilibrated with a 0.02 M tris-HCl buffer solution, pH = 7.4. The enzyme destabilase was eluted at a gradient NaCl 0.05-0.20 M (Fig. 1). Desalting is carried out by gel filtration with Superose-12. The position of the peak corresponding to destabilase is determined by the amidolytic activity of the fraction (substrate L - γ - Glu - pNA), which corresponds to 3.10 cat/mg (Fig. 2).

The method makes it possible to purify the enzyme 2400 times in respect of the initial extract. The yield of protein is 0.1% of the initial protein mixture. The homogeneity of the preparation is confirmed by the electrophoresis method in a gel of polyacrylamide.

### Example 2.

Medicinal leeches, starved for not less than four months, are comminuted and extracted with a physiological solution. The extract is applied to a column with agarose 6B with immobilized thereon antibodies to destabilase, equilibrated with a 0.02 M tris-HCl buffer solution, pH = 7.2. The destabilase is eluted with 0.2 M NaCl dissolved in a 0.02 M tris-HCl buffer solution, pH = 7.2. Fractions with amidolytic activity (substrate ε - γ - Glu - pNa) are combined and passed through a column with DEAE-sephadex A-L50, equilibrated with a 0.02 M tris-HCl buffer solution, pH = 7.2. The obtained fractions are applied to a column with CM-cellulose and eluted with a 0.02 M tris-HCl buffer solution, pH = 6.5 in a NaCl gradient from 0.1 to 0.3 M.

Fractions having amidolytic activity are combined, desalted by gel filtration through sephadex G-25.

As a result purification by 2500 times is achieved. The final amidolytic activity of destabilase (substrate L - γ - Glu - pNA) is 7.10 - 9 cat/mg.

The yield of protein is 0.12% of the initial content of the protein mixture. The homogeneity of the enzyme is confirmed by the method of electrophoresis in gel of polyacrylamide.

As a result of a study of the primary structure characterized by a high content of cysteine residues, the physicochemical, kinetic properties of the new thrombolytic enzyme--destabilase--the following characteristics have been obtained which make it possible to identify it.

UV-spectrum. There is a characteristic maximum of absorption at λ = 278 nm.

Molecular mass. The molecular mass of the monomer--destabilase--is 12600 ±300 Da. It is determined by the method of highly effective gel filtration on a column of Superose-12 (0.02 M tris-HCl, pH = 7.0) and gradient electrophoresis in 4-16% PAAG with 1% SDS.

Isopeptidase activity. The isopeptidase activity is determined by the capability of the enzyme to hydrolyze isopeptide bonds in a natural substrate--a product of limited proteolysis of stabilized fibrin--in a dimer of its D-fragment (D-D-dimer) in which isopeptide bonds between γ-γ-links of fibrin are maintained (Thromb. Res., 71, 1993, Elsevier Science Ltd., US, pp. 241-244). With this in mind, 0.1-2.0 µg of the enzyme are incubated with 50 µg of D-D-dimer in a 0.02 M tris-HCl buffer solution, pH = 8.0, during 10-70 hours. The total volume of the incubation mixture is 100 µl. As a result of the reaction, hydrolysis of the isopeptide bonds in the D-D-dimer and the formation of D-monomers are observed, and their accumulation is tracked using the electrophoresis method in PAAG. It has been established that the new protein causes solution of clots of stabilized fibrin, hydrolyzes γ - Glu - ε - Lys - isopeptide bonds in the dimer of fragment D and in the diisopeptide γ - Glu - ε - Lys.

Amidolytic activity. The amidolytic activity of the enzyme is determined by its capability of hydrolyzing an amide bond in γ - Glu - pNA with the formation of a nitroaniline by the method of Baskova I.P. et al (Biokhimiya, 55, 1990, Nauka, Moscow, pp. 674-679). In order to do this, 10-100 µl of a solution of the enzyme are added to a sample comprising 0.02 M tris-HCl of a buffer solution, pH = 8.0, and 0.05 mg/ml of γ - Glu - pNA in a total volume of 0.5 ml, and are incubated for 5-30 min at 25°C. The reaction is tracked spectrophotometrically at wavelength of 405 nm.

It has been established that the new enzyme hydrolyzes amide bonds in γ - Glu - dansylcadaverine, and in low molecular substrates of trypsin and chymotrypsin, comprising basic or hydrophobic amino acid in position P1 (Blood Coagulation and Fibrinolysis, 2, 1990, Rapid Communications of Oxford Ltd., GB, pp. 167-172).

Thermal stability. The enzyme retains activity at 22-37°C during 80 hours, at 7°C during 15 days, at 75°C during 15 min.

Range of pH stability. Activity is saved at pH from 1.5 to 8.5 (at 37°C during 7 days, substrate γ - Glu - pNA).

Primary structure. In order to identify the primary structure, the enzyme is chromatographed on a reverse-phase column with Agmapoke RP-300 (4.5 x 100) in a gradient of acetonitrile from 0 to 60% for 1 min. The N-terminal sequence of destabilase is determined. The 41st amino acid residues are identified. Threonine is determined as the N-terminal amino acid. Then CNBr-hydrolysis of destabilase is carried out and the obtained mixture is again chromatographed on the same column under the same conditions. As a result four peptide fragments are obtained. The amino acid sequence of the first fragment is fully determined, it consists of 25 amino acid residues, has a threonine residue at the N-terminal.

For the second fragment, the amino acid sequence of 28 amino acids from the N-terminal, with aspartic acid at the NH₂-terminal, is determined.

For the third fragment, the amino acid sequence of 24 amino acid residues from the N-terminal, with aspartic acid at the NH₂-terminal, is determined.

For the fourth fragment, 10 amino acid residues from the N-terminal and 5 amino acid residues from the C-terminal are determined. Glycine is the N-terminal amino acid.

Thrombolytic activity. The thrombolytic activity of the enzyme destabilase was analyzed in experiments on animals (rats with a body weight of 200 g). The formation of thrombi was stimulated in the jugular vein of the animals by administration of glass-activated blood serum with subsequent stasis in a section of the jugular vein (Circulation, 20, 1959, American Heart Association, US, pp. 864-867). The thrombus thus formed is fixed by applying a ligature to a corresponding section of the vein. Control animals received an intravenous injection of 0.2 ml of a physiological solution, while the experimental animals received the same volume of a solution of destabilase comprising 0.72 mg of protein. Thrombolysis was observed to 85% 67 hours after administration of the preparation, and to 100% after 137 hours after the administration. In the control group of animals, spontaneous thrombolysis to 17% is observed after 67 hours after administration of the physiological solution, and to 23% after 137 hours after administration (Blood coagulation and Fibrinolysis, 2, 1990, Rapid Communications of Oxford Ltd., GB, pp. 167-172).

### Industrial Applicability

The invention may be used as a thrombolytic biologically active preparation for clinical and experimental medicine.

SEQUENCE LISTING

(1) GENERAL INFORMATION

(i) APPLICANT
Eugene D. Sverdlov
Isolda P. Baskova
Lyudmila L. Zavalova
Sergey A. Lukyanov
Ekaterina V. Barsova
Ekaterina A. Bogdanova

(ii) TITLE OF INVENTION:
Thrombolytic Enzyme and Method of its Preparation

(iii) NUMBER OF SEQUENCES: 10

(iv) CORRESPONDENCE ADDRESS:
(A) ADDRESSEE: Eugene D.Sverdlov
(B) STREET: Miklukho-Maklaja, 16/10
(C) CITY: Moscow
(D) STATE: Russia
(E) COUNTRY: Russian Federation
(F) ZIP: 117871

(v) COMPUTER READABLE FORM:
(A) MEDIUM TYPE:DISKETTE, 3.50 INC, 1,44 Mb
(B) COMPUTER: IBM PC COMPATIBLE
(C) OPERATING SYSTEM: MS-DOS
(D) SOFTWARE; Wordperfect 5.1

(vi) CURRENT APPLICATION DATA:
(A) APPLICATION NUMBER: 95/00102
(B) FILLING DATA: 25 - May - 1995
(C) CLASSIFICATION: A61K 35/62, 37/02, ICI-6

(vii) PRIOR APPLICATION DATA:
(A) APPLICATION NUMBER: SU-94043698
(B) FILLING DATA: 23 - December -1994

(viii) ATTORNEY/AGENT INFORMATION:
(A) NAME:
(B) REGISTRATION NUMBER:
(C) REFERENCE/DOCKET NUMBER:

(ix) TELECOMMUNICATION INFORMATION:
(A) TELEPHONE: 095-330-65-29
(B) TELEFAX: 095-330-65-38

(2) INFORMATION FOR SEQ.ID NO:1:

(i) SEQUENCE CHARACTERISTICS
(A) LENGTH: 25 amino acids
(B) TYPE: amino acid
(D) TOPOLOGY: unknown

(ii) MOLECULAR TYPE
(A) DESCRIPTION: peptide

(v) FRAGMENT TYPE: N-terminal fragment

(xi) SEQUENCE DESCRIPTION: SEQ. ID NO: 1 :

Thr Val Pro Ser Asp Cys Leu Arg Cys Ile Cys Gln Val Glu Gly
1               5                   10                  15
Cys Asn Asn Glu Ile Gly Arg Cys Gly Met
                20                  25

(2) INFORMATION FOR SEQ. ID NO:2:

(i) SEQUENCE CHARACTERISTICS
(A) LENGTH: 28 amino acids
(B) TYPE: amino acid
(D) TOPOLOGY: unknown

(ii) MOLECULAR TYPE
(A) DESCRIPTION: peptide

(v) FRAGMENT TYPE: internal fragment

(xi) SEQUENCE DESCRIPTION: SEQ. ID NO: 2 :

Asp Ala Gly Ser Leu Ser Cys Gly Pro Tyr Gln Ile Lys Glu Pro
1               5                   10                  15
Tyr Trp Ile Asp Cys Gly Arg Pro Gly Gly Tyr Gln
20

(2) INFORMATION FOR SEQ. ID NO:3:

(1) SEQUENCE CHARACTERISTICS
(A) LENGTH: 24 amino acids
(B) TYPE: amino acid
(D) TOPOLOGY: unknown

(ii) MOLECULAR TYPE
(A) DESCRIPTION: peptide

(v) FRAGMENT TYPE: internal fragment

(xi) SEQUENCE DESCRIPTION: SEQ. 1D NO: 3 :

Asp Arg Tyr Ala Pro Pro Cys Thr Gly Gly Arg Gln Pro Thr Cys
1               5                   10                  15
Gln Asp Tyr Ala Lys Ile His Asn Met
                20

(2) INFORMATION FOR SEQ. ID NO 4:

(1) SEQUENCE CHARACTERISTICS
(A) LENGTH: 10 amino acids
(B) TYPE: amino acid
(D) TOPOLOGY: unknown

(ii) MOLECULAR TYPE
(A) DESCRIPTION: peptide

(v) FRAGMENT TYPE: internal fragment

(xi) SEQUENCE DESCRIPTION: SEQ. 1D NO: 4 :

Gly Pro Asn Gly Cys Gln Ser Leu Asn Asn
1               5                   10

(2) INFORMATION FOR SEQ. ID NO:5:

(i) SEQUENCE CHARACTERISTICS
(A) LENGTH: 162 base pairs

(B) TYPE: nucleic acid
(C) STRANDEDNESS: double
(D) TOPOLOGY: unkn
(v) FRAGMENT TYPE: internal fragment

(xi) SEQUENCE DESCRIPTION: SEQ.ID NO:5:

ATG AAT TAC 9
Met Asn Tyr
1
GTT ATC TTT GTG GTC TTA GTG GCA CTT TAC GTC ATC GAC GTA GCG 54
Val Ile Phe Val Val Leu Val Ala Leu Tyr Val Ile Asp Val Ala
    5                   10                  15
AAG TGC ACC GTT CCA TCC GAC TGC TTG AGT TGC ATT TGC GAG GTA 99
Lys Cys Thr Val Pro Ser Asp Cys Leu Ser Cys Ile Cys Glu Val
    20                  25                  30
GAG GGA TGT GAC AAA GAG ATT GGA AGG TGC GGC GAT GAC GCA GGA 144
Glu Gly Cys Asp Lys Glu Ile Gly Arg Cys Gly Asp Asp Ala Gly
    35                  40                  45
AGT CTG AGC TGT GGT CCT ... 162
Ser Leu Ser Cys Gly Pro ...
50

1 - 60 nucleotide - signal peptide
61 -162 nucleotide - structural part

(2) INFORMATION FOR SEQ. ID NO:6:

(i) SEQUENCE CHARACTERISTICS
(A) LENGTH: 408 base pairs

(B) TYPE: nucleic acid
(C) STRANDEDNESS: double
(D) TOPOLOGY: unknown

(v) FRAGMENT TYPE: internal fragment

(xi) SEQUENCE DESCRIPTION: SEQ.ID NO:6:

ATG ATC ATT GCA ATT 15
Met Ile Ile Ala Ile
1               5
TAT GTT TCC CTA GCT CTT CTA ATC GCC TCT GTC GAG GTG AAT AGC 60
Tyr Val Ser Leu Ala Leu Leu Ile Ala Ser Val Glu Val Asn Ser
                10                  15                  20
CAA TTC ACT GAT TCT TGC CTT CGG TGT ATT TGC AAG GTG GAA GGA 105
Gln Phe Thr Asp Ser Cys Leu Arg Cys Ile Cys Lys Val Glu Gly
                25                  30                  35
TGT GAC AGT CAA ATC GGA AAA TGT GGA ATG GAT GTT GGA AGC TTG 150
Cys Asp Ser Gln Ile Gly Lys Cys Gly Met Asp Val Gly Ser Leu
                40                  45                  50
AGT TGC GGA CCA TAC CAG ATT AAG AAA CCG TAC TGG ATT GAT TGT 195
Ser Cys Gly Pro Tyr Gln Ile Lys Lys Pro Tyr Trp Ile Asp Cys
                55                  60                  65
GGA AAA CCA GGG GGA GGT TAC GAA TCA TGC ACA AAA AAT AAA GCC 240
Gly Lys Pro Gly Gly Gly Tyr Glu Ser Cys Thr Lys Asn Lys Ala
                70                  75                  80
TGT TCA GAG ACT TGT GTG AGA GCT TAC ATG AAG AGG TAT GGA ACC 285
Cys Ser Glu Thr Cys Val Arg Ala Tyr Met Lys Arg Tyr Gly Thr
                85                  90                  95
TTC TGC ACA GGT GGA CGA ACG CCA ACC TGC CAG GAT TAT GCT AGG 330
Phe Cys Thr Gly Gly Arg Thr Pro Thr Cys Gln Asp Tyr Ala Arg
                100                 105                 110
ATT CAT ACC GGT GGA CCA CGC GGT TGC AAG AGT TCT GCT ACT GTT 375
Ile His Asn Gly Gly Pro Arg Gly Cys Lys Ser Ser Ala Thr Val
                115                 120                 125
GGT TAC TGG AAC AAG GTA CAG AAA TGT TTG AGA 408
Gly Tyr Trp Asn Lys Val Gln Lys Cys Leu Arg
                130                 135

1 - 60 nucleotide - signal peptide
61-408 nucleotide - structural part

(2) INFORMATION FOR SEQ.ID NO:7:

(i) SEQUENCE CHARACTERISTICS
(A) LENGTH: 115 residues
(B) TYPE: amino acid
(D) TOPOLOGY: unknown

(ii) MOLECULE TYPE:
(A) DESCRIPTION: protein

(xi) SEQUENCE DESCRIPTION : SEQ. ID NO:7:

Thr Val Pro Ser Asp Cys Leu Arg Cys Ile Cys Gln Val Glu Gly
1               5                   10                  15
Cys Asn Asn Glu Ile Gly Arg Cys Gly Met Asp Ala Gly Ser Leu
                20                  25                  30
Ser Cys Gly Pro Tyr Gln Ile Lys Glu Pro Tyr Trp Ile Asp Cys
                35                  40                  45
Gly Arg Pro Gly Gly Gly Tyr Gln Gln Cys Thr Lys Glu Lys Ala
                50                  55                  60
Cys Ser Glu Thr Cys Val His Ala Tyr Met Asp Arg Tyr Ala Arg
                65                  70                  75
Arg Cys Thr Gly Gly Arg Gln Pro Thr Cys Gln Asp Tyr Ala Lys
                80                  85                  90
Ile Hys Asn Met Gly Pro Asn Gly Cys Gln Ser Ser Asn Asn Hys
95 100 105
Tyr Trp Asp Asn Val Arg Arg Cys Leu Gly
110 115

(2) INFORMATION FOR SEQ. ID NO:8:

(i) SEQUENCE CHARACTERISTICS
(A). LENGTH: 568 base pairs

(B) TYPE: nucleic acid
(C) STRANDEDNESS: double

(D) TOPOLOGY: unknown

(xi) SEQUENCE DESCRIPTION: SEQ.1D NO:8:

C TTG AGC TGA ACT GAC AAT CTT AAT ATC TCA CTG ATG AAT TAC 43
Met Asn Tyr
1
GTT ATC TTT GTG GTC TTA GTG GCA CTT TAC GTC ATC GAC GTA GCG 88
Val Ile Phe Val Val Leu Val Ala Leu Tyr Val Ile Asp Val Ala
    5                   10                  15
AAG TGC ACC GTT CCA TCC GAC TGC TTG AGT TGC ATT TGC GAG GTA 133
Lys Cys Thr Val Pro Ser Asp Cys Leu Ser Cys Ile Cys Glu Val
    20                  25                  30
GAG GGA TGT GAC AAA GAG ATT GGA AGG TGC GGC GAT GAC GCA GGA 178
Glu Gly Cys Asp Lys Glu Ile Gly Arg Cys Gly Asp Asp Ala Gly
    35                  40                  45
AGT CTG AGC TGT GGT CCT TAC CAG ATC AAG GAG CCC TAC TGG ATT 223
Ser Leu Ser Cys Gly Pro Tyr Gln Ile Lys Glu Pro Tyr Trp Ile
    50                  55                  60
GAC TGT GGA AGT CCA GGA GCA GGA TAC CAG GAG TGC ACT AAG GAG 268
Asp Cys Gly Ser Pro Gly Ala Gly Tyr Gln Glu Cys Thr Lys Glu
    65                  70                  75
AAG GCA TGT TCC GAA ACG TGT GTC CAC GCT TAC ATG GAC AGG TAT 313
Lys Ala Cys Ser Glu Thr Cys Val His Ala Tyr Met Asp Arg Tyr
    80                  85                  90
GCC ACA AGG TGT ACT CGA GGA CGC CAA CCG ACC TGC CAA GAC TAC 358
Ala Thr Arg Cys Thr Arg Gly Arg Glu Pro Thr Cys Gln Asp Tyr
    95                  100                 105
GCC AAA ATT CAC AAC ATG GGA CCG AAC GGG TGC AGA CGT ACG AGC 403
Ala Lys Ile His Asn Met Gly Pro Asn Gly Cys Arg Arg Thr Ser
    110                 115                 120
AAC ACC TAC TGG AAC AAA GCC AAT GCG TGT CTG AAC TGA ACA AGA 448
Asn Thr Tyr Trp Asn Lys Ala Asn Ala Cys Leu Asn *
125 130
CAT TAT CGT CAG CTT CAG TCT GCC ATC TTT AAA GAT GG? ?GC TCT 493
CAA ATT CTA AAT TGT AAA GAG ATT TCT ACA GAC AGA ATT ATT AGT 538
TGA ATT TTT AAT AAA TTA TAT TAA AAT TGT 568

I-34 nucleic base - non-coding 5'region
35-94 nucleic base - signal peptide
95-442 nucleic base - coding sequence of the mature protein
443-568 nucleic base - 3'non-coding region
* - stop codon

(2) INFORMATION FOR SEQ. ID NO:9:

(i) SEQUENCE CHARACTERISTICS
(A) LENGTH: 630 base pairs
(B) TYPE: nucleic acid
(C) STRANDEDNESS: double
(D) TOPOLOGY: unknown

(xi) SEQUENCE DESCRIPTION : SEQ. ID NO:9:

AG TAA TGA AAA TTC ACC TTT CGG ACA AGG ATG ATC ATT GCA ATT 44
Met Ile Ile Ala Ile
1               5
TAT GTT TCC CTA GCT CTT CTA ATC GCC TCT GTC GAG GTG AAT AGC 89
Tyr Val Ser Leu Ala Leu Leu Ile Ala Ser Val Glu Val Asn Ser
                10                  15                  20
CAA TTC ACT GAT TCT TGC CTT CGG TGT ATT TGC AAG GTG GAA GGA 134
Gln Phe Thr Asp Ser Cys Leu Arg Cys Ile Cys Lys Val Glu Gly
                25                  30                  35
TGT GAC AGT CAA ATC GGA AAA TGT GGA ATG GAT GTT GGA AGC TTG 179
Cys Asp Ser Gln Ile Gly Lys Cys Gly Met Asp Val Gly Ser Leu
                40                  45                  50
AGT TGC GGA CCA TAC CAG ATT AAG AAA CCG TAC TGG ATT GAT TGT 224
Ser Cys Gly Pro Tyr Gln Ile Lys Lys Pro Tyr Trp Ile Asp Cys
                55                  60                  65
GGA AAA CCA GGG GGA GGT TAC GAA TCA TGC ACA AAA AAT AAA GCC 269
Gly Lys Pro Gly Gly Gly Tyr Glu Ser Cys Thr Lys Asn Lys Ala
                70                  75                  80
TGT TCA GAG ACT TGT GTG AGA GCT TAC ATG AAG AGG TAT GGA ACC 314
Cys Ser Glu Thr Cys Val Arg Ala Tyr Met Lys Arg Tyr Gly Thr
                85                  90                  95
TTC TGC ACA GGT GGA CGA ACG CCA ACC TGC CAG GAT TAT GCT AGG 359
Phe Cys Thr Gly Gly Arg Thr Pro Thr Cys Gln Asp Tyr Ala Arg
                100                 105                 110
ATT CAT ACC GGT GGA CCA CGC GGT TGC AAG AGT TCT GCT ACT GTT 404
Ile His Asn Gly Gly Pro Arg Gly Cys Lys Ser Ser Ala Thr Val
                115                 120                 125
GGT TAC TGG AAC AAG GTA CAG AAA TGT TTG AGA TGA ATT CGA AAT 449
Gly Tyr Trp Asn Lys Val Gln Lys Cys Leu Arg *
                130                 135
CTT TGA GTA GC? CCT GTC TTA CAT TTG AAA GGC CTT TTA ATT CAA 494
AAT TAT TTT GGG AAT G?A ATG ATT TTA AAC ATT TAT TTG AAA TTA 539
TTC ?GA AAT AGA AAC AAC TAT AAA TTG CTC CAA GAA TTG TAT AAT 584
CAT GAA GTG TTT GAA AGC TGT TTT CTG AAA TAA ACT TCC CAT AAA 629
T 630

1- 30 nucleic base -non-coding 5'region
31- 89 nucleic base -signal peptide
90-440 nucleic base -coding sequence of the mature protein
441-630 nucleic base -3'non-coding region
* - stop codo

(2) INFORMATION FOR SEQ. ID NO:10:

(i) SEQUENCE CHARACTERISTICS
(A) LENGTH: 559 base pairs
(B) TYPE: nucleic acid
(C) STRANDEDNESS: double
(D) TOPOLOGY: unknown

(xi) SEQUENCE DESCRIPTION : SEQ. ID NO:10:

GA ACT GGC AAT CTT AAT ATC TCA CTA ATG AAT TAC GTT ATC TTT 44
Met Asn Tyr Val Ile Phe
1               5
GTG GTC TTA GTG GCA CTT TAC GTC ATC GAC GTA GCG AAG TGC ACC 89
Val Val Leu Val Ala Leu Tyr Val Ile Asp Val Ala Lys Cys Thr
            10                  15                  20
GTC CCA TCC GAT TGC TTG AGG TGC ATT TGC CAG GTA GAG GGA TGT 134
Val Pro Ser Asp Cys Leu Arg Cys Ile Cys Gln Val Glu Gly Cys
            25                  30                  35
AAC AAT GAG ATT GGA AGG TGC GGC ATG GAC GCA GGA AGT CTG AGC 179
Asn Asn Glu Ile Gly Arg Cys Gly Met Asp Ala Gly Ser Leu Ser
            40                  45                  50
TGT GGT CCT TAC CAG ATC AAG GAG CCC TAC AGG ATT GAC TGT TGA 224
Cys Gly Pro Tyr Gln Ile Lys Glu Pro Tyr Trp Ile Asp Cys Gly
            55                  60                  65
AGG CCA GGA GGA GGA TAC CAG CAG TGC ACG AAG GAG AAG GCA TGT 269
Arg Pro Gly Gly Gly Tyr Gln Gln Cys Thr Lys Glu Lys Ala Cys
            70                  75                  80
TCT GAA AGG TGT GTC CAC GCT TAC ATG GAC AGG TAT GCC AGA AGG 314
Ser Glu Arg Cys Val His Ala Tyr Met Asp Arg Tyr Ala Arg Arg
            85                  90                  95
TGT ACT GGA GGA CGC CAA CCG ACC TGC CAA GAC TAC GCC AAA ATT 359
Cys Thr Gly Gly Arg Gln Pro Thr Cys Gln Asp Tyr Ala Lys Ile
            100                 105                 110
CAC AAC ATG GGA CCG AAC GGA TGC CAA TCT TCA AAG AAC CAC TAC 404
His Asn Met Gly Pro Asn Gly Cys Gln Ser Ser Asn Asn His Tyr
            115                 120                 125
TGG GAT AAT GTC AGG AGA TGT TTG GGC TGA AGA AAG AAG GAG GAA 449
Trp Asp Asn Val Arg Arg Cys Leu Gly *
            130                 135
CAA CAT TGC CTC AAG GTC GGC CAT TTT AAA GAT GGC TGC TGT TGA 494
TGT ATC AAA TTC TAA ATT AAA GAA AGG XCA TTT TTA AAT TGA ATA 539
AAT ACC AAA TGA TAA ATG AA 559

1-26 nucleotides - 5'noncoding region
27-85 nucleotides - signal peptide
86-431 nucleotides - structural part
432 - 559 nucleotides - 3'noncoding region
* - stop codon

## Claims

1. A thrombolytic enzyme capable of hydrolyzing exo- and endo - ε - (γ - Glu) - Lys - isopeptide bonds in synthetic substrates, in stabilized fibrin and in its D-D-fragment, characterized by a partial amino acid sequence with a structure of a corresponding thereto gene and two additional genes from that same family, and distinguished by the presence of residues of glycine in positions 15, 21, 24, 28, 33, 46, 49, 50 in the primary structure of the protein.

2. A method of preparing a trombolytic enzyme according to claim 1 from medicinal leech salivary gland secretion, or preparations comprising that secretion, characterized in that affinity chromatography on antibodies to native enzyme which are immobilized on a solid carrier is carried out, followed by subsequent purification of the enzyme by ion-exchange chromatography and gel filtration.
